# EUROPÄISCHE PATENTSCHRIFT

(11) **EP 3 270 841 B1**
(45) Veröffentlichungstag und Bekanntmachung des Hinweises auf die Patenterteilung: **31.07.2019**
(21) Anmeldenummer: 16710936.2
(22) Anmeldetag: 04.03.2016
(51) Int. Cl.: A61F 13/00, A61F 13/42, A61K 9/70, D02G 3/44, D03D 15/02

(54) **TEXTILES FLÄCHENGEBILDE ZUR AUFLAGE AUF DIE HAUT UND/ODER EINE WUNDE EINES PATIENTEN SOWIE TRANSDERMALES PFLASTER UND ANORDNUNG AUS EINEM TRANSDERMALEN PFLASTER UND EINER AUSWERTEINHEIT**
TEXTILE FABRIC FOR PLACING ON THE SKIN AND/OR A WOUND OF A PATIENT, AND TRANSDERMAL PATCH AND ARRANGEMENT CONSISTING OF A TRANSDERMAL PATCH AND AN EVALUATION UNIT
STRUCTURE TEXTILE PLATE POUR L'APPLICATION SUR LA PEAU ET/OU UNE PLAIE D'UN PATIENT AINSI QUE PATCH TRANSDERMIQUE ET DISPOSITIF CONSTITUÉ D'UN PATCH TRANSDERMIQUE ET D'UNE UNITÉ D'ÉVALUATION

(30) Priorität: 16.03.2015 DE 102015003254
(43) Veröffentlichungstag der Anmeldung: 24.01.2018
(73) Patentinhaber: Fresenius Medical Care Deutschland GmbH, 61352 Bad Homburg (DE)
(72) Erfinder: HEPPE, John, 66606 St. Wendel (DE)
(74) Vertreter: Oppermann, Frank
(86) Internationale Anmeldenummer: PCT/EP2016/054708
(87) Internationale Veröffentlichungsnummer: WO 2016/146410

(56) Entgegenhaltungen:
- EP-A2- 1 997 952
- WO-A1-2015/022671
- US-A1- 2013 053 754
- US-A1- 2014 012 199
- US-B1- 6 283 938

## Beschreibung

Die Erfindung betrifft ein textiles Flächengebilde zur Auflage auf die Haut und/oder eine Wunde eines Patienten mit einer der Haut bzw. Wunde abgewandten Oberseite und einer der Haut bzw. Wunde zugewandten Unterseite, an der das textile Flächengebilde eine für Feuchtigkeit undurchlässige Barriere aufweist, wobei das textile Flächengebilde aus nichtleitfähigen Kettfäden und nicht leitfähigen Schussfäden sowie leitfähigen Kettfäden und leitfähigen Schussfäden gebildet ist, die derart angeordnet sind, dass in dem textilen Flächengebilde eine elektrisch leitende Struktur aus leitfähigen Kett- und/oder Schussfäden ausgebildet ist. Darüber hinaus betrifft die Erfindung ein transdermales Pflaster zum Applizieren eines in einem Wirkstoffreservoir enthaltenden Wirkstoffes auf die Haut und/oder Wunde des Patienten mit einem derartigen textilen Flächengebilde sowie eine Anordnung aus einem transdermalen Pflaster und einer Auswerteinheit zur Erfassung der in dem Wirkstoffreservoir des transdermalen Pflasters enthaltenden Menge an Wirkstoff.

Aus der WO 2011/116943 ist ein Feuchtigkeitssensor zur Überwachung eines Gefäßzugangs bekannt, der als ein Gewebe aus nichtleitfähigen Kett- und Schussfäden sowie leitfähigen Kett- und Schussfäden ausgebildet ist. Die leitfähigen Kett- und Schussfäden bilden eine elektrisch leitende Struktur mit Anschlussleitungen, die Anschlusskontakte zum Anschluss einer Auswerteinheit aufweisen. Die Auswerteinheit misst den elektrischen Widerstand des zwischen den Anschlusskontakten befindlichen Abschnitts des Gewebes. Wenn das Gewebe mit Flüssigkeit benetzt wird, verändert sich dessen elektrischer Widerstand.

Die textilen Feuchtigkeitssensoren finden zur Überwachung eines Zugangs zu einem Patienten Verwendung, mit dem über eine Schlauchleitung eine Flüssigkeit einem Patienten zugeführt und/oder eine Flüssigkeit von dem Patienten abgeführt wird, insbesondere zur Überwachung des Gefäßzugangs bei einer extrakorporalen Blutbehandlung. Zur Überwachung des Gefäßzugangs werden die textilen Feuchtigkeitssensoren an der Punktionsstelle auf die Haut des Patienten aufgelegt.

Die WO 2010/091852 beschreibt einen Feuchtigkeitssensor aus einem saugfähigen Material mit einer elektrisch leitenden Struktur. Die der Haut des Patienten zugewandte Unterseite des Feuchtigkeitssensors ist mit einer für Flüssigkeit undurchlässigen Barriere vollständig bedeckt. Dadurch wird verhindert, dass Feuchtigkeit bzw. Schweiß von der Haut des Patienten an die elektrisch leitende Struktur gelangen kann.

Zur Applikation eines Wirkstoffes sind Wirkstoff-Pflaster bekannt, die auf die Haut des Patienten aufgeklebt werden. Diese Wirkstoffpflaster, die auch als transdermale Pflaster bezeichnet werden, weisen ein Wirkstoffreservoir auf, in dem eine bestimmte Menge an Wirkstoff enthalten ist. Für die Applikation des Wirkstoffes muss das Wirkstoffpflaster an der Unterseite zumindest abschnittsweise für Flüssigkeit durchlässig sein, wodurch aber zwangsläufig auch Schweiß von der Haut des Patienten in das Wirkstoffpflaster gelangen kann.

Ein transdermales Pflaster ist aus der US 6 283 938 B1 bekannt. Das transdermale Pflaster weist ein textiles Flächengebilde (Pad) auf, in dem sich ein Wirkstoffreservoir befindet, das über eine permeable Membran mit der Haut des Patienten in Kontakt kommen kann. Die Durchlässigkeit der permeablen Membran wird von einem Biosensor gesteuert, der unterhalb der Membran angeordnet ist. Die Funktion des transdermalen Pflasters setzt voraus, dass sich die Membran über den gesamten Bereich der Unterseite des Pads erstreckt

Die US 2014/0012199 A1 beschreibt ein Pad mit einer elektrisch leitenden Struktur aus leitfähigen Kett- und Schussfäden. Die Kett- und Schussfäden bilden ein Gewebe, das mit einem Trägermaterial kaschiert ist, das unter Temperatureinwirkung eine bleibende Verformung behält und nach der Verformung federelastisch ist, um ein Befestigungselement in der Art eines Clips auszubilden. Das Trägermaterial kann beispielsweise eine Kunststofffolie sein.

Der Erfindung liegt die allgemeine Aufgabe zugrunde, ein textiles Flächengebilde zur Auflage auf die Haut und/oder eine Wunde eines Patienten zu schaffen, das eine Messung von Feuchtigkeit, insbesondere Schweiß von der Haut des Patienten, erlaubt. Eine Aufgabe der Erfindung ist auch, den Einfluss von Schweiß von der Haut des Patienten auf ein Messergebnis zu verringern. Im Speziellen liegt der Erfindung die Aufgabe zugrunde, ein transdermales Pflaster zu schaffen, das die Bestimmung der in dem Wirkstoffreservoir enthaltenden Restmenge an Wirkstoff erlaubt, wobei der Einfluss von Schweiß von der Haut des Patienten auf das Messergebnis verringert ist.

Die Lösung dieser Aufgabe erfolgt erfindungsgemäß mit den Merkmalen des Patentanspruchs 1. Die Gegenstände der Unteransprüche betreffen vorteilhafte Ausführungsformen der Erfindung.

Das erfindungsgemäße textile Flächengebilde zur Auflage auf die Haut und/oder eine Wunde eines Patienten ist aus nicht-leitfähigen Kettfäden und nicht leitfähigen Schussfäden sowie leitfähigen Kettfäden und/oder leitfähigen Schussfäden gebildet, die derart angeordnet sind, dass in dem textilen Flächengebilde eine elektrisch leitende Struktur aus leitfähigen Kett- und/oder Schussfäden ausgebildet ist. Das textile Flächengebilde weist an der Unterseite eine für Feuchtigkeit undurchlässige Barriere auf, in der mindestens eine Aussparung vorgesehen ist. In dem Bereich der mindestens einen Aussparung der Barriere sind leitfähige Kett- und/oder Schussfäden angeordnet. Da die leitfähigen Kett- und/oder Schussfäden in dem Bereich der Aussparung mit Feuchtigkeit von der Haut des Patienten in Kontakt kommen, können die leitfähigen Kett- und/oder Schussfäden eine elektrisch leitende Struktur für eine Feuchtigkeitsmessung bilden. Mit der Feuchtigkeitsmessung ist insbesondere die Messung von Schweiß von der Haut des Patienten möglich.

In diesem Zusammenhang wird unter einer Aussparung beispielsweise eine Ausstanzung (punch-out), eine Unterbrechung, eine Weglassung, eine Öffnung, ein Durchbruch, ein Schlitz, ein Loch, eine Lochung, ein Absatz (recess), eine Lücke (gap) oder eine Perforation verstanden. Die Aussparung kann durch Ausstanzen oder Ausschneiden insbesondere mittels eines Lasers erfolgen.

Für die Auflage des textilen Flächengebildes auf eine Wunde muss die Aussparung größer als die Wunde sein, so dass das textile Flächengebilde auf der Haut, die die Wunde umgibt, kleben kann.

Eine bevorzugte Ausführungsform des textilen Flächengebildes sieht die Erfassung von Messwerten mit einem ersten Sensor und die Erfassung von Messwerten mit einem zweiten Sensor vor, wobei den ersten und zweiten Sensor leitfähige Kett- und/oder Schussfäden der elektrisch leitenden Struktur bilden. Die den ersten Sensor bildenden elektrisch leitfähigen Kett- und/oder Schussfäden sind in dem Bereich einer ersten Aussparung der für Feuchtigkeit undurchlässigen Barriere und die den zweiten Sensor bildenden elektrisch leitfähigen Kett- und/oder Schussfäden in dem Bereich einer zweiten Aussparung der Barriere angeordnet. Der erste und/oder zweite Sensor können auch jeweils nur aus mindestens zwei Kettfäden oder aus mindestens zwei Schussfäden gebildet werden.

Der erste Sensor und der zweite Sensor können unterschiedlich ausgebildet sein und der Erfassung von unterschiedlichen Messgrößen dienen. Die Sensoren können als resistive Sensoren ausgebildet sein. Aber auch eine Ausbildung als kapazitive Sensoren ist möglich. Die resistiven Sensoren können von jeweils zwei im Abstand zueinander angeordneten Kettfäden oder Schussfäden gebildet werden, wobei der elektrische Widerstand bzw. die Leitfähigkeit des Abschnitts des Gewebes zwischen den benachbarten Kett- bzw. Schussfäden gemessen werden kann. Die leitfähigen Kett- und/oder Schussfäden der elektrisch leitenden Struktur können auch die Anschlussleitungen für den ersten und zweiten Sensor bilden, wobei die Enden der Anschlussleitungen des ersten und zweiten Sensors als Anschlusskontakte ausgebildet sein können, die vorzugsweise an einem Abschnitt des textilen Flächengebildes vorgesehen sind, das als eine Anschlusslasche ausgebildet ist.

Eine bevorzugte Ausführungsform der Erfindung sieht vor, dass einer der beiden Sensoren ein Feuchtigkeitssensor ist, der die Messung von Feuchtigkeit an der Haut des Patienten erlaubt, während der andere Sensor der Erfassung eines beliebigen Zustandes oder einer beliebigen Größe dienen kann. Der Messung der Feuchtigkeit erlaubt dann die Kompensation des Einflusses von Feuchtigkeit, insbesondere Schweiß, an der Haut des Patienten auf die Erfassung des beliebigen Zustandes oder der beliebigen Größe.

Eine besonders bevorzugte Ausführungsform der Erfindung, bei der die Vorteile der Erfindung besonderes zum Tragen kommen, liegt in der Verwendung des textilen Flächengebildes als transdermales Pflaster, das über ein Wirkstoffreservoir verfügt, in dem eine bestimmte Menge eines Wirkstoffes enthalten ist, der über die Haut oder direkt in die Wunde des Patienten appliziert wird, wenn das transdermale Pflaster auf der Haut bzw. der Wunde aufliegt. Diese Ausführungsform erlaubt die Bestimmung der in dem Wirkstoffreservoir enthaltenden Menge an Wirkstoff, wobei der Einfluss von Schweiß von der Haut des Patienten auf das Messergebnis verringert ist.

Das Wirkstoffreservoir ist im Bereich der leitfähigen Kett- und/oder Schussfäden des im Bereich der ersten Aussparung angeordneten ersten Sensors angeordnet. Durch die erste Aussparung kommt die Haut bzw. Wunde des Patienten mit dem Wirkstoff in Kontakt, so dass der Wirkstoff appliziert werden kann. Der erste Sensor im Bereich der Aussparung kommt sowohl mit dem Wirkstoffreservoir als auch mit Schweiß von der Haut in Kontakt. Der zweite Sensor ist im Bereich der zweiten Aussparung angeordnet, so dass dieser nur mit Schweiß von der Haut des Patienten in Kontakt kommen kann.

Für die Applikation einer ausreichenden Menge an Wirkstoff nimmt die erste Aussparung in dem textilen Flächengebilde vorzugsweise eine größere Fläche als die zweite Aussparung ein. Die Aussparungen können unterschiedlich ausgebildet sein, beispielsweise rechteckförmig, kreisförmig etc. sein. Die Form der Aussparung für die Applikation des Wirkstoffes kann an die Größe und Form einer Wunde oder eines zu versorgenden Bereichs der Haut angepasst sein.

Wenn die für den Wirkstoff vorgesehene erste Aussparung in der für Feuchtigkeit undurchlässigen Barriere (erster Sensor) in deren Haupterstreckungsebene eine andere Fläche bzw. Größe aufweist als die für die Messung von Schweiß vorgesehene Aussparung in der für Feuchtigkeit undurchlässigen Barriere (zweiter Sensor), kann in der Auswerteinheit eine Korrektur mit einem Korrekturfaktor vorgenommen werden, die den Einfluss der unterschiedlichen Fläche bzw. Größe der ersten und der zweiten Aussparung kompensiert.

Zur Erfassung der in dem Wirkstoffreservoir des Wirkstoffpflasters enthaltenden Menge an Wirkstoff kann das erfindungsgemäße Wirkstoffpflaster an eine Auswerteinheit angeschlossen werden.

Die erfindungsgemäße Auswerteinheit weist eine Einrichtung zum Erfassen der Messwerte des ersten und zweiten Sensors auf, die derart konfiguriert ist, dass aus den Messwerten des ersten Sensors ein mit der in dem Wirkstoffreservoir befindlichen Menge an Wirkstoff und mit Feuchtigkeit auf der Haut korrelierender erster Messwert und aus den Messwerten des zweiten Sensors ein mit Feuchtigkeit auf der Haut korrelierender zweiter Messwert gewonnen wird.

Eine bevorzugte Ausführungsform sieht vor, dass die Auswerteinheit derart konfiguriert ist, dass der erste Messwert mit einem aus dem zweiten Messwert gewonnenen Korrekturwert korrigiert wird. Dadurch kann der Einfluss von Schweiß auf der Haut des Patienten auf die Bestimmung der in dem Wirkstoffreservoir enthaltenden Menge an Wirkstoff kompensiert werden.

Die Auswerteinheit ist vorzugsweise derart konfiguriert, dass auf der Grundlage der Auswertung der Messwerte des ersten und zweiten Sensors ein Signal erzeugt wird, dass signalisiert, dass in dem Wirkstoffreservoir noch Wirkstoff enthalten ist, und/oder ein Signal erzeugt wird, dass signalisiert, dass in dem Wirkstoffreservoir Wirkstoff nicht mehr enthalten ist. Diese Signale können Alarmsignale sein, die einen akustischen und/oder optischen und/oder taktilen Alarm auslösen oder Steuersignale, mit denen Eingriffe in eine Maschinensteuerung vorgenommen werden können.

Die Auswertung der Messwerte der beiden Sensoren kann auf verschiedenen Verfahren beruhen. Bei einer bevorzugten Ausführungsform ist die Auswerteinheit derart konfiguriert, dass der erste Messwert mit dem zweiten Messwert verglichen wird, wobei ein Signal erzeugt wird, das signalisiert, dass in dem Wirkstoffreservoir kein Wirkstoff enthalten ist, wenn der erste Messwert gleich dem zweiten Messwert ist oder der Betrag der Differenz zwischen dem ersten und zweiten Messwert kleiner als ein vorgegebener Grenzwert ist. Die Messwerte beider Sensoren sind in diesem Fall zumindest annährend gleich, da beide Sensoren nur den Schweiß von der Haut erfassen können. Dabei wird davon ausgegangen, dass die beiden Aussparungen so dicht nebeneinander liegen, dass in diesen Bereichen die Schweißproduktion zumindest annähernd gleich ist.

Eine besonders bevorzugte Ausführungsform sieht vor, dass die Kett- und/oder Schussfäden der elektrisch leitenden Struktur derart angeordnet sind, dass in dem textilen Flächengebilde vier im Abstand zueinander angeordnete Leiterbahnabschnitte ausgebildet sind, wobei zwei Leiterbahnabschnitte im Abstand zueinander im Bereich der ersten Aussparung und zwei Leiterbahnabschnitte im Abstand zueinander im Bereich der zweiten Aussparung angeordnet sind. Jeweils zwei Leiterbahnabschnitte, die von Abschnitten der leitfähigen Fäden gebildet werden, bilden eine Messstelle (Sensor). Zu jeder Kontaktfläche führt eine Anschlussleitung, wobei an den Enden der Anschlussleitungen Anschlusskontakte ausgebildet sind. Die Kett- und/oder Schussfäden der elektrisch leitenden Struktur sind weiterhin derart angeordnet, dass eine den ersten und dritten Leiterbahnabschnitt miteinander verbindende Verbindungsleitung ausgebildet ist. Die Auswerteinheit weist bei dieser Ausführungsform eine Einrichtung auf, die derart konfiguriert ist, dass der elektrische Widerstand zwischen dem ersten Anschlusskontakt und dem zweiten Anschlusskontakt, der elektrische Widerstand zwischen dem ersten Anschlusskontakt und dem dritten Anschlusskontakt, der elektrische Widerstand zwischen dem ersten Anschlusskontakt und dem vierten Anschlusskontakt, der elektrische Widerstand zwischen dem zweiten Anschlusskontakt und dem dritten Anschlusskontakt, der elektrische Widerstand zwischen dem zweiten Anschlusskontakt und dem vierten Anschlusskontakt und der elektrische Widerstand zwischen dem dritten und vierten Anschlusskontakt gemessen wird. Die Messung dieser elektrischen Widerstände erlaubt unter Verwendung einer geeigneten Auswertroutine die Überwachung der Wirkstoffmenge unabhängig von der Schweißproduktion. Es versteht sich von selbst, dass anstelle des Widerstandes auch die Leitfähigkeit gemessen werden kann.

Eine weitere bevorzugte Verwendung kann in der Kompensation des Einflusses von Schweiß auf die Überwachung eines Zugangs zu einem Patienten liegen, mit dem über eine Schlauchleitung eine Flüssigkeit einem Patienten zugeführt und/oder eine Flüssigkeit von dem Patienten abgeführt wird, insbesondere zur Überwachung des Gefäßzugangs bei einer extrakorporalen Blutbehandlung. Bei dieser Ausführungsform eines textilen Flächengebildes zur Überwachung eines Gefäßzugangs ist der erste Sensor ein Feuchtigkeitssensor zur Messung von Blut.

Im Folgenden wird ein Ausführungsbeispiel der Erfindung unter Bezugnahme auf die Figuren näher erläutert.

Es zeigen:
- Fig. 1: ein transdermales Pflaster in vereinfachter schematischer Darstellung,
- Fig. 2: einen Schnitt durch das transdermale Pflaster von Fig. 1 in vergrößerter Darstellung,
- Fig. 3: ein elektrisches Ersatzschaltbild der elektrisch leitenden Struktur des transdermalen Pflasters von Fig. 1 und
- Fig. 4: ein elektrisches Ersatzschaltbild für ein Ausführungsbeispiel.

Nachfolgend wird als ein Ausführungsbeispiel für die Verwendung des erfindungsgemäßen textilen Flächengebildes ein textiles transdermales Pflaster beschrieben, das über ein Wirkstoffreservoir verfügt.

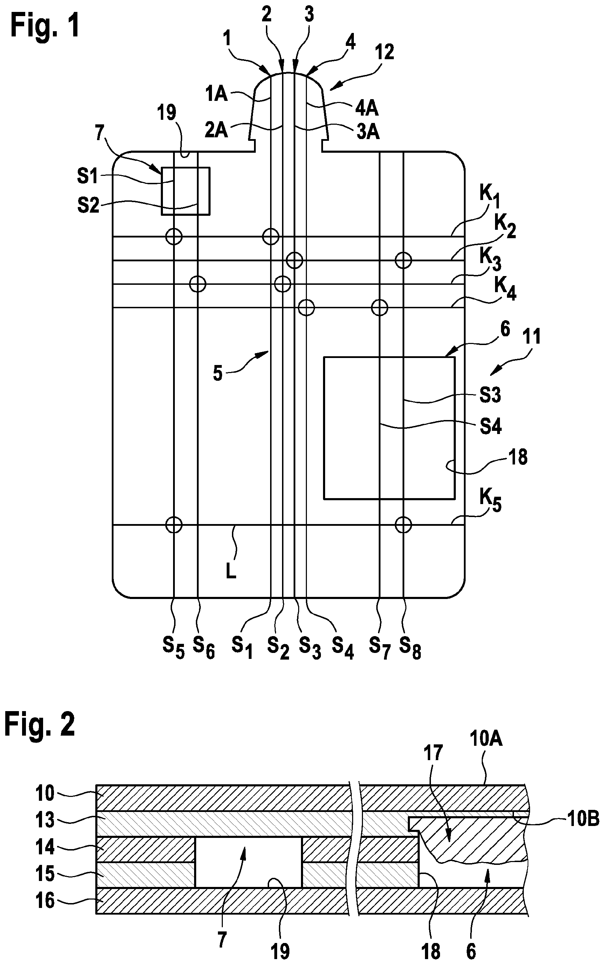

Fig. 1 zeigt eine vereinfachte schematische Darstellung eines Ausführungsbeispiels des erfindungsgemäßen Wirkstoffpflasters in der Draufsicht. Fig. 2 zeigt einen Schnitt durch das Wirkstoffpflaster in vergrößerter Darstellung.

Das transdermales Pflaster weist ein mehrlagiges Gewebe 10 auf, das in der WO 2011/116943 im Einzelnen beschrieben ist. Das Mehrlagengewebe 10 besteht aus elektrisch leitfähigen und elektrisch nicht leitfähigen Kett- und Schussfäden. Die elektrisch leitfähigen und elektrisch nicht leitfähigen Kett- und Schussfäden sind derart angeordnet, dass das Gewebe eine der Haut des Patienten zugewandte untere Lage, eine mittlere Lage und eine der Haut des Patienten abgewandte obere Lage aufweist. Die Aufteilung des Gewebes in mehrere Lagen dient aber nur dem besseren Verständnis des Gewebeaufbaus, da sich in der Praxis die Lagen nicht genau voneinander trennen lassen. Eine Struktur von elektrischen Leiterbahnen wird in der unteren und mittleren Lage des Gewebes dadurch gebildet, dass die elektrisch leitfähigen Kett- und/oder Schussfäden an den Kreuzungspunkten derart angeordnet werden, dass sie entweder elektrisch leitend miteinander verbunden sind oder elektrisch voneinander isoliert sind. Die obere Lage des Gewebes kann als Isolationsschicht gegen Berührung ausgebildet sein.

Das Wirkstoffpflaster weist einen rechteckförmigen Abschnitt 11 mit zwei Längsseiten und zwei Schmalseiten auf. An einer der beiden Schmal- oder Längsseiten ist vorzugsweise mittig eine Anschlusslasche 12 zum Anschluss einer Auswerteinheit angeordnet. Anstelle einer einzigen Anschlusslasche können auch zwei getrennte Anschlusslaschen vorgesehen sein, wobei mit der einen Anschlusslasche eine elektrische Verbindung zwischen dem ersten Sensor (Wirkstoff) und der Auswerteinheit und mit der anderen Anschlusslasche eine elektrische Verbindung zwischen dem zweiten Sensor (Schweiß) und der Auswerteinheit hergestellt werden kann.

Die elektrisch leitfähigen Kettfäden sind mit "K" und die elektrisch leitfähigen Schussfäden mit "S" bezeichnet. Die elektrisch nicht leitfähigen Kett- und Schussfäden sind in Fig. 1 nicht dargestellt.

Von der Anschlusslasche 12 führen vier leitfähige Schussfäden S (S₁, S2, S₃, S₄) zu der gegenüberliegenden Seite des Gewebes, während zu beiden Seiten der Anschlusslasche jeweils zwei leitfähige Schussfäden S (S₅, S₆, S₇, Sg) zu der gegenüberliegenden Seite führen. Auf der Hälfte des rechteckförmigen Abschnitts, die der Anschlusslasche 12 zugewandt ist, erstrecken sich zwischen den gegenüberliegenden Seiten vier leitfähige Kettfäden K (K₁, K₂, K₃, K₄) und auf der der Anschlusslasche abgewandten Hälfte erstreckt sich ein leitfähiger Kettfaden K (K₅). Die Kreuzungspunkte (Kontaktstellen) der leitfähigen Kett- und Schussfäden K, S, an denen die Kett- und Schussfäden K, S elektrisch kontaktieren, sind mit einem Kreis gekennzeichnet.

Die Enden der leitfähigen Kett- und Schussfäden K, S an der Anschlusslasche 12 sind als Anschlusskontakte 1, 2, 3, 4 ausgebildet, an denen ein in Fig. 1 nicht dargestellter elektrischer Anschlussteil einer Auswerteinheit angeschlossen werden kann.

Die leitfähigen Kett- und Schussfäden K, S bilden eine elektrisch leitende Struktur 5 in dem Gewebe 10. Die Kett- und Schussfäden K, S sind in dem Gewebe derart angeordnet, dass die elektrisch leitende Struktur 5 einen ersten resistiven Sensor 6 und einen zweiten resistiven Sensor 7 umfasst, die jeweils von elektrisch leitenden Abschnitten S3, S4 bzw. S1, S2 zweier parallel verlaufender Schussfäden gebildet werden. Dabei bilden jeweils zwei benachbarte leitende Abschnitte der Schussfäden elektrische Leiterbahnabschnitte einer Messstelle (Sensor).

Bei einer speziellen alternativen Ausführungsform umfassen die Messstellen jeweils einen Schussfaden und einen diesen in der Draufsicht des Flächengebildes kreuzenden Kettfaden, wobei der Kettfaden und der Schussfaden an der Kreuzungsstelle im dreidimensionalen Gewebe voneinander isoliert sind. Bei einer Befeuchtung der Kreuzungsstelle wird diese elektrisch leitend.

Das Gewebe 10 aus nicht leitfähigen Kettfäden und nicht leitfähigen Schussfäden sowie leitfähigen Kettfäden und leitfähigen Schussfäden weist eine der Haut des Patienten abgewandte Oberseite 10A auf. An der dem Patienten zugewandten Unterseite 10B ist das Gewebe mit einer Klebeschicht 13 versehen, an deren Unterseite sich eine für Flüssigkeit undurchlässige Barriere 14 befindet. Die Unterseite der Barriere ist mit einer Klebeschicht 15 versehen, an der eine abziehbare Schicht 16, beispielsweise eine Silikonfolie, haftet, die vor dem Aufbringen des Wirkstoffpflaster auf die Haut des Patienten abgezogen wird.

Das Gewebe 10 weist ein nur andeutungsweise dargestelltes Wirkstoffreservoir 17 auf, in dem eine bestimmte Menge eines Wirkstoffs enthalten ist. Ein Abschnitt des Gewebes ist beispielsweise mit dem Wirkstoff getränkt.

In der mit der Klebeschicht 15 versehenen Barriere 14 ist unterhalb der beiden parallelen Abschnitte der Schussfäden S3, S4 des ersten resistiven Sensors 6 eine erste Aussparung 18 und unterhalb der beiden parallelen Abschnitte der Schussfäden S1, S2 des zweiten resistiven Sensors 7 eine zweite Aussparung 19 vorgesehen. Die erste Aussparung 18 hat eine größere Fläche als die zweite Aussparung 19. Der Abschnitt des Gewebes mit dem Wirkstoffreservoir 17 befindet sich oberhalb der ersten Aussparung 18, so dass nach Abziehen der Silikonfolie 16, die auch als Liner bezeichnet wird, der Wirkstoff durch die erste Aussparung 18 mit der Haut und/oder Wunde des Patienten in Kontakt kommen kann.

Das Wirkstoffreservoir 17 erstreckt sich aus der Klebeschicht 13 in die Aussparung 18 der für Flüssigkeit undurchlässigen Barriere 14 hinein und kann die Haut und/oder Wunde des Patienten berühren, wenn das Silikonpapier (Liner) abgezogen und das Pflaster mit der (dünnen) Klebeschicht 15 auf die Haut des Patienten geklebt wird.

Der elektrische Widerstand des Abschnitts des Gewebes, der zwischen den Abschnitten der Schussfäden S3, S4 liegt, die den ersten Sensor 6 bilden, ist von der Menge des in dem Wirkstoffreservoir 17 enthaltenden Wirkstoffs abhängig. Daher erlaubt die Messung des Widerstandes die Überwachung der Wirkstoffinenge in dem Reservoir. Der elektrische Widerstand kann aber auch von Schweiß verändert werden, der durch die Aussparung 18 mit dem ersten Sensor 6 in Kontakt kommen kann. Dadurch kann das Messergebnis verfälscht werden. Das Wirkstoffpflaster sieht daher den zweiten Sensor 7 vor. Der Widerstand des Abschnitts des Gewebes, der zwischen den Abschnitten der Schussfäden S1, S2 liegt, die den zweiten Sensor 7 bilden, ist von der Feuchtigkeit der Haut des Patienten, d.h. der Menge des Schweißes, abhängig, der durch die zweite Aussparung 19 mit dem zweiten Sensor 7 in Kontakt kommt. Da der Wirkstoff nicht mit dem zweiten Sensor 7 in Kontakt kommt, wird mit dem zweiten Sensor nur die Menge des Schweißes auf der Haut gemessen, die eine Korrekturgröße für die gemessene Menge des Wirkstoffes darstellt.

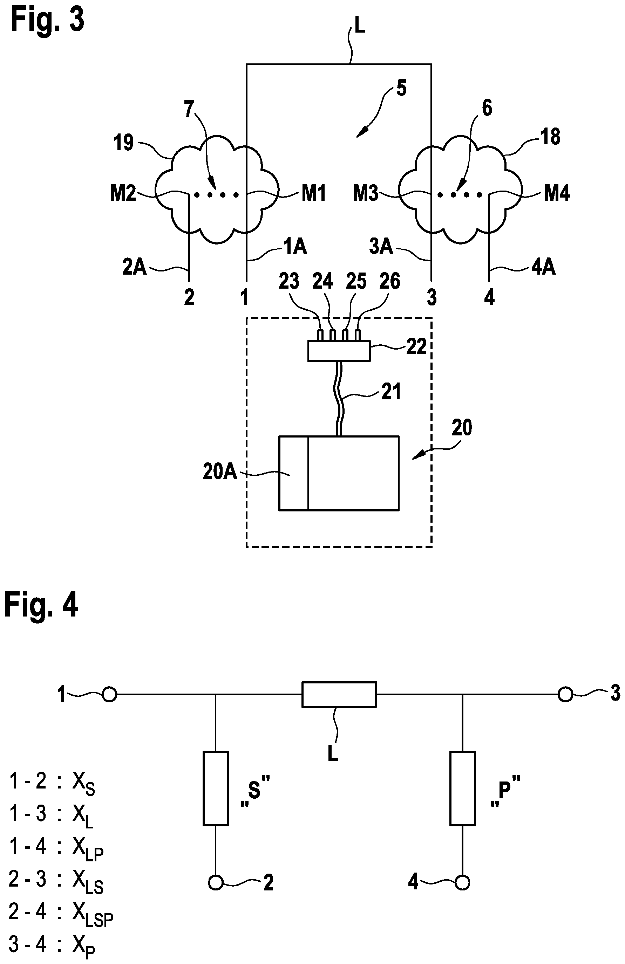

Fig. 3 zeigt ein elektrisches Ersatzschaltbild der elektrisch leitenden Struktur 5 des in den Figuren 1 und 2 dargestellten Wirkstoffpflasters. Die einander entsprechenden Teile sind in den Figuren mit den gleichen Bezugszeichen versehen. Die Leiterbahnabschnitte des ersten Sensors 6 und die Leiterbahnabschnitte des zweiten Sensors 7, die von den jeweiligen Abschnitten der Schussfäden gebildet werden, befinden sich an den Kontaktstellen zur Haut des Patienten in der ersten und zweiten Aussparung 18, 19 der Barriere 14. Damit ergeben sich in dem textilen Flächengebilde insgesamt vier im Abstand zueinander angeordnete Leiterbahnabschnitte M1, M2, M3, M4, wobei der dritte und vierte Leiterbahnabschnitt M3, M4 im Abstand zueinander im Bereich der ersten Aussparung 18 und der erste und zweite Leiterbahnabschnitt M1, M2 im Abstand zueinander im Bereich der zweiten Aussparung 19 angeordnet sind. Die beiden Leiterbahnabschnitte in der ersten Aussparung bilden den ersten Sensor, während die beiden Leiterbahnabschnitte in der zweiten Aussparung den zweiten Sensor bilden. Folglich werden die Sensoren von leitendenden Abschnitten der Kett- und/oder Schussfäden gebildet. Die elektrisch leitende Struktur 5 umfasst weiterhin eine zu dem ersten Leiterbahnabschnitt M1 führende erste Anschlussleitung 1A und eine zu dem zweiten Leiterbahnabschnitt M2 führende zweite Anschlussleitung 2A und eine zu dem dritten Leiterbahnabschnitt M3 führende dritte Anschlussleitung 3A und eine zu dem vierten Leiterbahnabschnitt M4 führende vierte Anschlussleitung 4A und eine den ersten und dritten Leiterbahnabschnitt M1, M3 miteinander verbindende Verbindungsleitung L, wobei an den Enden der Anschlussleitungen die Anschlusskontakte 1, 2, 3, 4 ausgebildet sind.

Der Widerstand xP des Gewebes zwischen den Leiterbahnabschnitten M3 und M4, d.h. der Messwert des ersten Sensors 6, ist proportional der Wirkstoff- und Schweißmenge, während der Widerstand xS des Gewebes zwischen den Leiterbahnabschnitten M1 und M2, d.h. der Messwert des zweiten Sensors 7, proportional der Schweißmenge ist. Die Verbindungsleitung L hat einen Widerstand xL. Der Widerstand der Anschlussleitungen 1A bis 4A wird vernachlässigt.

Der zwischen den Anschlusskontakten 1 und 2 gemessene Widerstand xS hängt von der Schweißmenge ab, während der zwischen den Anschlusskontakten 3 und 4 gemessene Widerstand xP von der Schweißmenge und der Wirkstoffmenge abhängt. Zwischen den Anschlusskontakten 1 und 3 wird der Widerstand xL der Verbindungsleitung L gemessen.

Der zwischen den Anschlusskontakten 1 und 4 gemessene Widerstand xLP ist vom Widerstand xL der Verbindungsleitung L und der Schweiß- und Wirkstoffmenge abhängig und der zwischen den Anschlusskontakten 2 und 3 gemessene Widerstand xLS ist vom Widerstand xL der Verbindungsleitung L und der Schweißmenge abhängig. Der zwischen den Anschlusskontakten 2 und 4 gemessene Widerstand xLSP ist vom Widerstand xL der Verbindungsleitung L, der Schweißmenge und der Wirkstoffmenge abhängig.

Wenn xP = xS ist, kann darauf geschlossen werden, dass der gemessene Widerstand nur auf den Schweiß zurückzuführen ist, d.h. die in dem Wirkstoffreservoir enthaltende Menge an Wirkstoff vollständig verbraucht ist. Auf den vollständigen Verbrauch des Wirkstoffs kann auch dann geschlossen werden, wenn xLSP = xL + 2 xS bzw. xLSP = xL + 2 xP ist (xS=xP). Wenn hingegen xLSP - xL - 2xS ≠ 0 ist, kann darauf geschlossen werden, dass in dem Wirkstoffreservoir noch Wirkstoff enthalten ist.

Für ein Ausführungsbeispiel eines textilen Flächengebildes ergeben sich beispielsweise die folgenden Werte für die elektrischen Widerstände, die auf Erfahrungswerten bei bekannten Fadensystemen beruhen.

| | **xS [Ω]** |
|---|---|
| MIN | 100 (Probe ist nass) |
| SOLL | 100000000 |
| MAX | 1000000000 (Probe ist trocken) (Variable) |

| | **xL[Ω]** |
|---|---|
| MIN | 30 |
| SOLL | 60 |
| MAX | 100 (Variable) |

| | **xLP[Ω]** |
|---|---|
| MIN | 130 |
| SOLL | 100060 |
| MAX | 1000000100 (Berechneter Wert) |

| | **xLS[Ω]** |
|---|---|
| MIN | 130 |
| SOLL | 100000060 |
| MAX | 1000000100 (Berechneter Wert) |

| | **xLSP[Ω]** |
|---|---|
| MIN | 230 |
| SOLL | 100100060 |
| MAX | 2000000100 (Berechneter Wert) |

| | **xP[Ω]** |
|---|---|
| MIN | 100 |
| SOLL | 100000 |
| MAX | 1000000000 |

Fig. 4 zeigt ein elektrisches Ersatzschaltbild für das Ausführungsbeispiel mit den Widerständen "S" (Schweiß) und "P" (Wirkstoff) sowie der L (Verbindungsleitung).

Für die Auswertung der Messergebnisse ist eine Auswerteinheit 20 vorgesehen, die ein Anschlusskabel 21 mit einem Anschlussteil 22 aufweist, das vier Anschlusskontakte 23, 24, 25, 26 hat, die an den Anschlusskontakten 1, 2, 3, 4 der Anschlusslasche 12 des Wirkstoffpflasters angeschlossen werden (Fig. 3). Die Auswerteinheit 20 weist eine Einrichtung auf, die derart konfiguriert ist, dass der elektrische Widerstand xS zwischen dem ersten Anschlusskontakt 1 und dem zweiten Anschlusskontakt 2, der elektrische Widerstand xL zwischen dem ersten Anschlusskontakt 1 und dem dritten Anschlusskontakt 3, der elektrische Widerstand xLP zwischen dem ersten Anschlusskontakt 1 und dem vierten Anschlusskontakt 4, der elektrische Widerstand xLS zwischen dem zweiten Anschlusskontakt 2 und dem dritten Anschlusskontakt 3, der elektrische Widerstand xLSP zwischen dem zweiten Anschlusskontakt 2 und dem vierten Anschlusskontakt 4 und der elektrische Widerstand xP zwischen dem dritten und vierten Anschlusskontakt 3, 4 gemessen wird.

Bei einem Ausführungsbeispiel ist die Auswerteinheit 20 derart konfiguriert, dass die Differenz xP - xS mit einem vorgegebenen Grenzwert verglichen wird, der auch Null sein kann (xP = xS). Wenn die Differenz xP - xS größer oder gleich dem Grenzwert ist, erzeugt die Auswerteinheit 20 ein Signal, das signalisiert, dass in dem Wirkstoffreservoir noch eine ausreichende Menge an Wirkstoff enthalten ist. Wenn die Differenz xP - xS hingegen kleiner als der Grenzwert ist, erzeugt die Auswerteinheit 20 ein Signal, das signalisiert, dass in dem Wirkstoffreservoir kein Wirkstoff mehr enthalten ist. Wenn xLSP - xL - 2 xS ≠ 0 ist, erzeugt die Auswerteinheit 20 ein Signal, das signalisiert, dass in dem Wirkstoffreservoir noch Wirkstoff enthalten ist.

Eine alternative Ausführungsform sieht die Messung der absoluten Menge an Wirkstoff mit einer Korrektur der Messwerte vor. Bei dieser Ausführungsform ist eine Funktion in einem Speicher 20A der Auswerteinheit 20 gespeichert, die die Abhängigkeit des mit dem ersten Sensor 6 gemessenen Widerstands von der Wirkstoffmenge beschreibt. Diese Funktion kann in Versuchen ermittelt werden. Die Auswerteinheit 20 ist derart konfiguriert, dass aus dem Widerstandswert, der mit dem ersten Sensor gemessen wird, ein Wert für die Wirkstoffmenge berechnet wird. Gleichzeitig wird mit dem zweiten Sensor 7 ein Maß für die Schweißmenge gemessen, wobei auch für die Abhängigkeit des Widerstands von der Schweißmenge eine empirisch ermittelte Funktion abgespeichert ist. In dem Speicher 20A der Auswerteinheit 20 ist darüber hinaus eine Korrekturfunktion gespeichert, mit der der zuvor berechnete Wert für die Wirkstoffmenge in Abhängigkeit von der gemessenen Schweißmenge korrigiert wird. Dadurch wird erreicht, dass sich ein Maß für die Wirkstoffmenge genau bestimmen lässt, das von dem Einfluss des Schweißes bereinigt ist. Diese Korrekturfunktion kann in Versuchen ermittelt werden.

## Patentansprüche

1. Textiles Flächengebilde zur Auflage auf die Haut und/oder eine Wunde eines Patienten mit einer der Haut und/oder Wunde abgewandten Oberseite und einer der Haut und/oder Wunde zugewandten Unterseite, an der das textile Flächengebilde eine für Feuchtigkeit undurchlässige Barriere (14) aufweist, wobei das textile Flächengebilde aus nicht-leitfähigen Kettfäden und nicht leitfähigen Schussfäden sowie leitfähigen Kettfäden und/oder leitfähigen Schussfäden gebildet ist, die derart angeordnet sind, dass in dem textilen Flächengebilde eine elektrisch leitende Struktur (5) aus leitfähigen Kett- und/oder Schussfäden (K, S) ausgebildet ist,
**dadurch gekennzeichnet,**
**dass** die für Feuchtigkeit undurchlässige Barriere (14) an der Unterseite des textilen Flächengebildes mindestens eine Aussparung (18, 19) aufweist, wobei in dem Bereich der Aussparung der für Feuchtigkeit undurchlässigen Barriere leitfähige Kett- und/oder Schussfäden (K, S) angeordnet sind, so dass die leitfähigen Kett- und/oder Schussfäden in dem Bereich der Aussparung mit Feuchtigkeit von der Haut des Patienten in Kontakt kommen können.

2. Textiles Flächengebilde nach Anspruch 1, **dadurch gekennzeichnet, dass** leitfähige Kett- und/oder Schussfäden (K, S) einen ersten Sensor (6) und leitfähige Kett- und/oder Schussfäden einen zweiten Sensor (7) bilden, wobei die den ersten Sensor bildenden leitfähigen Kett- und/oder Schussfäden in dem Bereich einer ersten Aussparung (18) der für Feuchtigkeit undurchlässigen Barriere (14) und die den zweiten Sensor (7) bildenden leitfähigen Kett- und/oder Schussfäden in dem Bereich einer zweiten Aussparung (19) der Barriere angeordnet sind.

3. Textiles Flächengebilde nach Anspruch 2, **dadurch gekennzeichnet, dass** der erste Sensor (6) und der zweite Sensor (7) als resistive Sensoren ausgebildet sind.

4. Textiles Flächengebilde nach Anspruch 2 oder 3, **dadurch gekennzeichnet, dass** der erste Sensor (6) und der zweite Sensor (7) von jeweils zwei im Abstand zueinander angeordneten Kettfäden oder Schussfäden (K, S) gebildet werden.

5. Textiles Flächengebilde nach einem der Ansprüche 2 bis 4, **dadurch gekennzeichnet, dass** leitfähige Kett- und/oder Schussfäden Anschlussleitungen (3A, 4A) für den ersten Sensor (6) und leitfähige Kett- und/oder Schussfäden Anschlussleitungen (1A, 2A) für den zweiten Sensor (7) bilden.

6. Textiles Flächengebilde nach Anspruch 5, **dadurch gekennzeichnet, dass** die Enden der Anschlussleitungen (3A, 4A) des ersten Sensors (6) als Anschlusskontakte (3, 4) und die Enden der Anschlussleitungen (1A, 2A) des zweiten Sensors (7) als Anschlusskontakte (1, 2) ausgebildet sind.

7. Textiles Flächengebilde nach Anspruch 6, **dadurch gekennzeichnet, dass** ein Abschnitt des textilen Flächengebildes als eine Anschlusslasche (12) ausgebildet ist, an der die Anschlusskontakte (1, 2, 3, 4) angeordnet sind.

8. Transdermales-Pflaster zum Applizieren eines Wirkstoffes auf die Haut und/oder Wunde eines Patienten mit einem textilen Flächengebilde nach einem der Ansprüche 2 bis 7 und einem Wirkstoffreservoir (17), **dadurch gekennzeichnet, dass** das Wirkstoffreservoir (17) im Bereich der leitfähigen Kett- und/oder Schussfäden (K, S) des im Bereich der ersten Aussparung (18) angeordneten ersten Sensors (6) angeordnet ist, so dass der erste Sensor mit dem Wirkstoffreservoir (17) und mit Feuchtigkeit von der Haut in Kontakt kommen kann.

9. Transdermales-Pflaster nach Anspruch 1, **dadurch gekennzeichnet, dass** die erste Aussparung (18) in dem textilen Flächengebilde eine größere Fläche als die zweite Aussparung (19) einnimmt.

10. Vorrichtung mit einem transdermalen Pflaster nach Anspruch 8 oder 9 und einer Auswerteinheit (20) zur Erfassung der in dem Wirkstoffreservoir enthaltenden Menge an Wirkstoff.

11. Vorrichtung nach Anspruch 10, **dadurch gekennzeichnet, dass** die Auswerteinheit (20) eine Einrichtung zum Erfassen der Messwerte des ersten Sensors (6) und zum Erfassen der Messwerte des zweiten Sensors (7) aufweist, die derart konfiguriert ist, dass aus den Messwerten des ersten Sensors (6) ein mit der in dem Wirkstoffreservoir (17) enthaltenden Menge an Wirkstoff und mit Feuchtigkeit auf der Haut korrelierender erster Messwert und aus den Messwerten des zweiten Sensors (7) ein mit der Feuchtigkeit auf der Haut korrelierender zweiter Messwert gewonnen wird.

12. Vorrichtung nach Anspruch 11, **dadurch gekennzeichnet, dass** die Auswerteinheit (20) derart konfiguriert ist, dass der erste Messwert mit einem aus dem zweiten Messwert gewonnenen Korrekturwert korrigiert wird.

13. Vorrichtung nach Anspruch 11 oder 12, **dadurch gekennzeichnet, dass** die Auswerteinheit (20) derart konfiguriert ist, dass auf der Grundlage der Auswertung der Messwerte des ersten und zweiten Sensors ein Signal erzeugt wird, dass signalisiert, dass in dem Wirkstoffreservoir (17) Wirkstoff enthalten ist, und/oder ein Signal erzeugt wird, dass signalisiert, dass in dem Wirkstoffreservoir (17) Wirkstoff nicht enthalten ist.

14. Vorrichtung nach Anspruch 12, **dadurch gekennzeichnet, dass** die Auswerteinheit (20) derart konfiguriert ist, dass der erste Messwert mit dem zweiten Messwert verglichen wird, wobei ein Signal erzeugt wird, das signalisiert, dass in dem Wirkstoffreservoir (17) ein Wirkstoff nicht enthalten ist, wenn der erste Messwert gleich dem zweiten Messwert ist oder die Differenz zwischen dem ersten und zweiten Messwert kleiner als ein vorgegebener Grenzwert ist.

15. Vorrichtung nach Anspruch 13 oder 14, **dadurch gekennzeichnet, dass**
die Kett- und/oder Schussfäden (K, S) der elektrisch leitenden Struktur (5) derart angeordnet sind, dass
in dem textilen Flächengebilde vier im Abstand zueinander angeordnete elektrische Leiterbahnabschnitte ausgebildet sind, wobei zwei Leiterbahnabschnitte (M3, M4) im Abstand zueinander im Bereich der ersten Aussparung (18) angeordnet sind und den ersten Sensor (6) bilden und zwei Leiterbahnabschnitte (M1, M2) im Abstand zueinander im Bereich der zweiten Aussparung (19) angeordnet sind und den zweiten Sensor (7) bilden, und
eine zu dem ersten Leiterbahnabschnitt (M1) führende erste Anschlussleitung (A1) und eine zu dem zweiten Leiterbahnabschnitt (M2) führende zweite Anschlussleitung (A2) und eine zu dem dritten Leiterbahnabschnitt (M3) führende dritte Anschlussleitung (A3) und eine zu dem vierten Leiterbahnabschnitt (M4) führende vierte Anschlussleitung (A4) und eine den ersten und dritten Leiterbahnabschnitt (M1, M3) miteinander verbindende Verbindungsleitung (L) ausgebildet sind, wobei an den Enden der Anschlussleitungen (A1, A2, A3, A4) Anschlusskontakte (1, 2, 3, 4) ausgebildet sind, und
dass die Auswerteinheit (18) eine Einrichtung aufweist, die derart konfiguriert ist, dass der elektrische Widerstand zwischen dem ersten Anschlusskontakt (1) und dem zweiten Anschlusskontakt (2), der elektrische Widerstand zwischen dem ersten Anschlusskontakt (1) und dem dritten Anschlusskontakt (3), der elektrische Widerstand zwischen dem ersten Anschlusskontakt (1) und dem vierten Anschlusskontakt (4), der elektrische Widerstand zwischen dem zweiten Anschlusskontakt (2) und dem dritten Anschlusskontakt (3), der elektrische Widerstand zwischen dem zweiten Anschlusskontakt (2) und dem vierten Anschlusskontakt (4) und der elektrische Widerstand zwischen dem dritten und vierten Anschlusskontakt (3, 4) gemessen wird.

## Claims

1. Textile fabric to be placed onto the skin and/or a wound of a patient, comprising a top side facing away from the skin and/or wound and an underside which faces the skin and/or wound and on which the textile fabric has a moisture-impermeable barrier (14), the textile fabric being formed from non-conductive warp threads and non-conductive weft threads and from conductive warp threads and/or conductive weft threads which are arranged such that an electrically conductive structure (5) consisting of conductive warp and/or weft threads (K, S) is formed in the textile fabric,
**characterised in that**
the moisture-impermeable barrier (14) on the underside of the textile fabric comprises at least one opening (18, 19), conductive warp and/or weft threads (K, S) being arranged in the region of the opening in the moisture-impermeable barrier such that the conductive warp and/or weft threads can come into contact with moisture from the patient's skin in the region of the opening.

2. Textile fabric according to claim 1, **characterised in that** conductive warp and/or weft threads (K, S) form a first sensor (6) and conductive warp and/or weft threads form a second sensor (7), the conductive warp and/or weft threads which form the first sensor being arranged in the region of a first opening (18) in the moisture-impermeable barrier (14) and the conductive warp and/or weft threads which form the second sensor (7) being arranged in the region of a second opening (19) in the barrier.

3. Textile fabric according to claim 2, **characterised in that** the first sensor (6) and the second sensor (7) are formed as resistive sensors.

4. Textile fabric according to either claim 2 or claim 3, **characterised in that** the first sensor (6) and the second sensor (7) are formed in each case by two warp threads or weft threads (K, S) which are arranged at a spacing from one another.

5. Textile fabric according to any of claims 2 to 4, **characterised in that** conductive warp and/or weft threads form connection lines (3A, 4A) for the first sensor (6) and conductive warp and/or weft threads form connection lines (1A, 2A) for the second sensor (7).

6. Textile fabric according to claim 5, **characterised in that** the ends of the connection lines (3A, 4A) of the first sensor (6) are formed as connection contacts (3, 4) and the ends of the connection lines (1A, 2A) of the second sensor (7) are formed as connection contacts (1, 2).

7. Textile fabric according to claim 6, **characterised in that** a portion of the textile fabric is formed as a connection tab (12), on which the connection contacts (1, 2, 3, 4) are arranged.

8. Transdermal patch for applying an active substance to the skin and/or wound of a patient, comprising a textile fabric according to any of claims 2 to 7 and an active substance reservoir (17), **characterised in that** the active substance reservoir (17) is arranged in the region of the conductive warp and/or weft threads (K, S) of the first sensor (6), which is arranged in the region of the first opening (18), such that the first sensor can come into contact with both the active substance reservoir (17) and moisture from the skin.

9. Transdermal patch according to claim 1, **characterised in that** the first opening (18) in the textile fabric occupies a larger surface area than the second opening (19).

10. Device comprising a transdermal patch according to either claim 8 or claim 9 and an evaluation unit (20) for detecting the amount of active substance in the active substance reservoir.

11. Device according to claim 10, **characterised in that** the evaluation unit (20) comprises an apparatus for acquiring the measured values from the first sensor (6) and for acquiring the measured values from the second sensor (7), which apparatus is configured to obtain, from the measured values from the first sensor (6), a first measured value which correlates with the amount of active substance in the active substance reservoir (17) and with moisture on the skin, and to obtain, from the measured values from the second sensor (7), a second measured value which correlates with the moisture on the skin.

12. Device according to claim 11, **characterised in that** the evaluation unit (20) is configured to correct the first measured value using a correction value obtained from the second measured value.

13. Device according to either claim 11 or claim 12, **characterised in that** the evaluation unit (20) is configured to generate a signal on the basis of the evaluation of the measured values from the first and second sensors, which signal indicates that there is active substance in the active substance reservoir (17), and/or to generate a signal which indicates that there is no active substance in the active substance reservoir (17).

14. Device according to claim 12, **characterised in that** the evaluation unit (20) is configured to compare the first measured value with the second measured value, a signal being generated which indicates that there is no active substance in the active substance reservoir (17) when the first measured value is the same as the second measured value or the difference between the first and second measured values is smaller than a predetermined limit value.

15. Device according to either claim 13 or claim 14, **characterised in that**
the warp and/or weft threads (K, S) of the electrically conductive structure (5) are arranged such that
four electric conductor track portions are arranged in the textile fabric at a spacing from one another, two conductor track portions (M3, M4) being arranged at a spacing from one another in the region of the first opening (18) and forming the first sensor (6), and two conductor track portions (M1, M2) being arranged at a spacing from one another in the region of the second opening (19) and forming the second sensor (7), and
a first connection line (A1) is formed leading to the first conductor track portion (M1), a second connection line (A2) is formed leading to the second conductor track portion (M2), a third connection line (A3) is formed leading to the third conductor track portion (M3), a fourth connection line (A4) is formed leading to the fourth conductor track portion (M4), and a connecting line (L) is formed interconnecting the first and third conductor track portions (M1, M3), connection contacts (1, 2, 3, 4) being formed at the ends of the connection lines (A1, A2, A3, A4), and
**in that** the evaluation unit (18) comprises an apparatus which is configured to measure the electrical resistance between the first connection contact (1) and the second connection contact (2), the electrical resistance between the first connection contact (1) and the third connection contact (3), the electrical resistance between the first connection contact (1) and the fourth connection contact (4), the electrical resistance between the second connection contact (2) and the third connection contact (3), the electrical resistance between the second connection contact (2) and the fourth connection contact (4), and the electrical resistance between the third and fourth connection contacts (3, 4).

## Revendications

1. Structure textile plate destinée à être appliquée sur la peau et/ou une plaie d'un patient avec une face supérieure opposée à la peau et/ou la plaie et une face inférieure tournée vers la peau et/ou la plaie, au niveau de laquelle la structure textile plate présente une barrière (14) ne laissant pas passer l'humidité, dans laquelle la structure textile plate est formée à partir de fils de chaîne non conducteurs et de fils de trame non conducteurs ainsi qu'à partir de fils de chaîne conducteurs et/ou de fils de trame conducteurs, qui sont disposés de telle manière qu'une structure (5) électriquement conductrice est réalisée dans la structure textile plate à partir de fils de chaîne et/ou de trame (K, S) conducteurs,
**caractérisée en ce**
**que** la barrière (14) ne laissant pas passer l'humidité présente au niveau de la face inférieure de la structure textile plate au moins un évidement (18, 19), dans laquelle des fils de chaîne et/ou de trame (K, S) conducteurs sont disposés dans la zone de l'évidement de la barrière ne laissant pas passer l'humidité si bien que les fils de chaîne et/ou de trame conducteurs peuvent venir en contact dans la zone de l'évidement avec de l'humidité provenant de la peau du patient.

2. Structure textile plate selon la revendication 1, **caractérisée en ce que** des fils de chaîne et/ou de trame (K, S) conducteurs forment un premier capteur (6) et des fils de chaîne et/ou de trame conducteurs forment un second capteur (7), dans laquelle les fils de chaîne et/ou de trame conducteurs formant le premier capteur sont disposés dans la zone d'un premier évidement (18) de la barrière (14) ne laissant pas passer l'humidité et les fils de chaîne et/ou de trame conducteurs formant le second capteur (7) sont disposés dans la zone d'un second évidement (19) de la barrière.

3. Structure textile plate selon la revendication 2, **caractérisée en ce que** le premier capteur (6) et le second capteur (7) sont réalisés sous la forme de capteurs résistifs.

4. Structure textile plate selon la revendication 2 ou 3, **caractérisée en ce que** le premier capteur (6) et le second capteur (7) sont formés par respectivement deux fils de chaîne ou fils de trame (K, S) disposés à distance l'un de l'autre.

5. Structure textile plate selon l'une quelconque des revendications 2 à 4, **caractérisée en ce que** des fils de chaîne et/ou de trame conducteurs forment des lignes de raccordement (3A, 4A) pour le premier capteur (6) et des fils de chaîne et/ou de trame conducteurs forment des lignes de raccordement (1A, 2A) pour le second capteur (7).

6. Structure textile plate selon la revendication 5, **caractérisée en ce que** les extrémités des lignes de raccordement (3A, 4A) du premier capteur (6) sont réalisées sous la forme de contacts de raccordement (3, 4) et les extrémités des lignes de raccordement (1A, 2A) du second capteur (7) sont réalisées sous la forme de contacts de raccordement (1, 2).

7. Structure textile plate selon la revendication 6, **caractérisée en ce qu'**une section de la structure textile plate est réalisée sous la forme d'une bride de raccordement (12), au niveau de laquelle les contacts de raccordement (1, 2, 3, 4) sont disposés.

8. Pansement transdermique servant à appliquer un principe actif sur la peau et/ou la plaie d'un patient avec une structure textile plate selon l'une quelconque des revendications 2 à 7 et un réservoir de principe actif (17), **caractérisé en ce que** le réservoir de principe actif (17) est disposé dans la zone des fils de chaîne et/ou de trame (K, S) conducteurs du premier capteur (6) disposé dans la zone du premier évidement (18) si bien que le premier capteur peut venir en contact avec le réservoir de principe actif (17) et avec de l'humidité provenant de la peau.

9. Pansement transdermique selon la revendication 1, **caractérisé en ce que** le premier évidement (18) occupe dans la structure textile plate une surface plus grande que le second évidement (19).

10. Dispositif avec un pansement transdermique selon la revendication 8 ou 9 et une unité d'évaluation (20) servant à détecter la quantité de principe actif contenue dans le réservoir de principe actif.

11. Dispositif selon la revendication 10, **caractérisé en ce que** l'unité d'évaluation (20) présente un système servant à détecter les valeurs de mesure du premier capteur (6) et servant à détecter les valeurs de mesure du second capteur (7), qui est configuré de telle manière qu'une quantité de principe actif contenue dans le réservoir de principe actif (17) et une première valeur de mesure en corrélation avec l'humidité sur la peau sont obtenues à partir des valeurs de mesure du premier capteur (6) et une seconde valeur de mesure en corrélation avec l'humidité sur la peau est obtenue à partir des valeurs de mesure du second capteur (7).

12. Dispositif selon la revendication 11, **caractérisé en ce que** l'unité d'évaluation (20) est configurée de telle manière que la première valeur de mesure est corrigée avec une valeur de correction obtenue à partir de la seconde valeur de mesure.

13. Dispositif selon la revendication 11 ou 12, **caractérisé en ce que** l'unité d'évaluation (20) est configurée de telle manière qu'est généré, sur la base de l'évaluation des valeurs de mesure du premier et du second capteur, un signal, qui indique que du principe actif est contenu dans le réservoir de principe actif (17), et/ou est généré un signal, qui indique qu'aucun principe actif n'est contenu dans le réservoir de principe actif (17).

14. Dispositif selon la revendication 12, **caractérisé en ce que** l'unité d'évaluation (20) est configurée de telle manière que la première valeur de mesure est comparée à la seconde valeur de mesure, dans lequel est généré un signal, qui indique qu'aucun principe actif n'est contenu dans le réservoir de principe actif (17) quand la première valeur de mesure est égale à la seconde valeur de mesure ou quand la différence entre la première et la seconde valeur de mesure est inférieure à une valeur limite prédéterminée.

15. Dispositif selon la revendication 13 ou 14, **caractérisé en ce que**
les fils de chaîne et/ou de trame (K, S) de la structure (5) électriquement conductrice sont disposés de telle manière que
quatre sections de piste conductrice électriques disposées à distance les unes des autres sont réalisées dans la structure textile plate, dans lequel deux sections de piste conductrice (M3, M4) sont disposées à distance l'une de l'autre dans la zone du premier évidement (18) et forment le premier capteur (6) et deux sections de piste conductrice (M1, M2) sont disposées à distance l'une de l'autre dans la zone du second évidement (19) et forment le second capteur (7), et
une première ligne de raccordement (A1) menant vers la première section de piste conductrice (M1) et une deuxième ligne de raccordement (A2) menant vers la deuxième section de piste conductrice (M2) et une troisième ligne de raccordement (A3) menant vers la troisième section de piste conductrice (M3) et une quatrième ligne de raccordement (A4) menant vers la quatrième section de piste conductrice (M4) et une ligne de liaison (L) reliant entre elles la première et la troisième section de piste conductrice (M1, M3) sont réalisées, dans lequel des contacts de raccordement (1, 2, 3, 4) sont réalisés au niveau des extrémités des lignes de raccordement (A1, A2, A3, A4), et
**que** l'unité d'évaluation (18) présente un système, qui est configuré de telle manière que la résistance électrique entre le premier contact de raccordement (1) et le deuxième contact de raccordement (2), la résistance électrique entre le premier contact de raccordement (1) et le troisième contact de raccordement (3), la résistance électrique entre le premier contact de raccordement (1) et le quatrième contact de raccordement (4), la résistance électrique entre le deuxième contact de raccordement (2) et le troisième contact de raccordement (3), la résistance électrique entre le deuxième contact de raccordement (2) et le quatrième contact de raccordement (4) et la résistance électrique entre le troisième et le quatrième contact de raccordement (3, 4) sont mesurées.
